Europäisches Patentamt

European Patent Office

Office européen des brevets

(19)

‖‖‖‖‖‖‖‖‖‖‖‖‖‖‖‖‖‖‖‖‖‖‖‖‖‖‖‖‖

(11) Numéro de publication : **0 414 608 B1**

# (12) FASCICULE DE BREVET EUROPEEN

(45) Date de publication du fascicule du brevet :
**10.08.94 Bulletin 94/32**

(51) Int. Cl.$^5$ : **A61K 7/48**

(21) Numéro de dépôt : **90402330.6**

(22) Date de dépôt : **22.08.90**

(54) **Nouvelles compositions cosmétiques contenant du chitosane et de la glucosamine.**

(30) Priorité : **23.08.89 FR 8911170**

(43) Date de publication de la demande :
**27.02.91 Bulletin 91/09**

(45) Mention de la délivrance du brevet :
**10.08.94 Bulletin 94/32**

(84) Etats contractants désignés :
**BE CH DE FR GB IT LI LU NL**

(56) Documents cités :
**WO-A-87/07618**
**US-A- 4 542 014**
**PATENT ABSTRACTS OF JAPAN, vol. 9, no.
108 (C-280)[1831], 15 mai 1985; & JP-A-60 01
110 (AJINOMOTO) 07-01-1985**

(73) Titulaire : **ROUSSEL-UCLAF**
**35, Boulevard des Invalides**
**F-75007 Paris (FR)**

(72) Inventeur : **Noel, Hugues**
**29, rue du Général Lherillier**
**F-95120 Ermont (FR)**

(74) Mandataire : **Bourgouin, André et al**
**Département des Brevets**
**ROUSSEL UCLAF**
**111, route de Noisy**
**B.P. no 9**
**F-93230 Romainville (FR)**

EP 0 414 608 B1

**EP 0 414 608 B1**

## Description

La présente invention concerne de nouvelles compositions cosmétiques contenant du chitosane et de la glucosamine.

L'invention a pour objet de nouvelles compositions cosmétiques destinées à traiter la couche cornée de l'épiderme, notamment en permettant simultanément une hydratation et une amélioration de l'état de surface de la peau de façon durable.

Le document WO-A-8701246 décrit des compositions renfermant du chitosane et de l'acide succinique.

Le document Patent Abstracts of Japan, Vol. 9, Nr. 108 (C-280) (1831), 11/5/85 décrit des compositions renfermant du chitosane et de la glucosamine.

De nombreux produits hydratants existent déjà.

Il existe également des produits destinés au conditionnement des kératines, obtenus par exemple par absorption de molécules renfermant un ou plusieurs groupements ammonium quaternaire.

Mais l'hydratation de la couche cornée par les humectants conventionnels s'accompagne d'une diminution des caractéristiques mécaniques telles qu'une diminution de l'élasticité ou une augmentation de l'extensibilité. Une augmentation de la perméabilité transcutanée peut également être observée.

Or il vient d'être découvert, et c'est l'objet de la présente invention, que ces effets peuvent être obtenus simultanément et modulés en fonction de l'état ou de la nature de la peau.

L'invention a ainsi pour objet de nouvelles compositions cosmétiques renfermant du chitosane, de la glucosamine et au moins un acide organique choisi parmi l'acide gluconique ou l'acide succinique, destinées notamment à hydrater et améliorer l'état de surface de l'épiderme.

Le chitosane ou chitine désacétylée peut être obtenu par hydrolyse de la chitine. La chitine est, après la cellulose, le polysaccharide le plus répandu dans la nature.

Dans le règne animal, la chitine est un élément structural important des ligaments de certains invertébrés tels que les insectes, les mollusques, les crustacés et les nématodes.

Dans le règne végétal, elle n'est signalée que dans les parois des champignons et de certaines algues chlorophycées.

Des références bibliographiques sur la chitine et le chitosane peuvent être citées telles que :
- Merck Index 10me éd. n° 2017,
- "Chitin" éd. MUZZARELLI R.A.A. Pergamon Press Oxford (1977)
- "Chitine, chitosane et dérivés, de nouvelles perspectives pour l'industrie" SENG J.M. Biofutur (9. 1988) p. 40-44.

L'association de chitosane (chimiquement proche des glycosaminoglycanes du derme) filmogène et substantif (fortement affine pour la peau, le cheveu ou l'ongle), de la glucosamine et d'un acide organique dans un produit cosmétique permet d'augmenter le taux d'hydratation de l'épiderme et de produire les effets classiques des agents tenseurs (protéines de haut poids moléculaire, résines anioniques) et conditionneurs souvent incompatibles.

L'invention a plus particulièrement pour objet de nouvelles compositions cosmétiques telles que définies ci-dessus caractérisées en ce que :
- le chitosane est compris entre 0,2 et 5 %,
- la glucosamine est comprise entre 0,2 et 5 %,
- l'acide gluconique est compris entre 0,2 et 5 %,
- l'acide succinique est compris entre 0,1 et 5 %.

Ces pourcentages sont ceux des produits dans la composition cosmétique finale correspondante.

L'invention a notamment pour objet de nouvelles compositions cosmétiques telles que définies ci-dessus caractérisées en ce que le chitosane qu'elles renferment comprend 0 à 30 % de groupements aminés acétylés ainsi que de nouvelles compositions cosmétiques telles que définies ci-dessus caractérisées en ce que le chitosane qu'elles renferment provient de carapaces de crabe et comprend moins de 15 % de groupements aminés acétylés et de préférence 0,1 à 5 %.

Le taux de désacétylation de la chitine dans le chitosane doit préférentiellement être supérieur à 80 % pour permettre une solubilisation totale en milieu faiblement acide.

L'invention a également pour objet de nouvelles compositions cosmétiques telles que définies ci-dessus caractérisées en ce qu'elles renferment une ou plusieurs substances à une concentration suffisante pour amener le pH de la préparation entre 3 et 6,5.

Les compositions cosmétiques telles que définies ci-dessus peuvent être éventuellement combinées à une ou plusieurs substances, dont le rôle principal est de moduler le pH de la composition.

Les substances que l'on peut employer pour adapter le pH des compositions peuvent être choisies parmi les acides acétique, lactique, tartrique, malique, mucique, hydrobutyrique, glycolique, hydroxycaproïque, hy-

2

droxyoctanoïque, aspartique, glutamique, pyrrolidonecarboxylique, chlorhydrique et nitrique ou encore les hydroxydes de sodium, potassium et magnésium, le diméthylaminoéthanol, le diéthylaminoéthanol, la lysine, l'arginine, la citrulline et l'ornithine.

Les compositions ainsi obtenues sont hygroscopiques, leur déshydratation complète nécessite l'emploi d'un lyophilisateur.

Suivant la composition du mélange, l'hygroscopicité est variable et permet l'adaptation aux différents types de peau.

L'invention concerne aussi notamment un procédé de préparation des compositions telles que définies ci-dessus caractérisées en ce que l'on prépare d'abord une solution acide d'un pH inférieur à 3,5 en mélangeant le chitosane à au moins un des acides gluconique ou succinique, puis ajoute les autres constituants de la composition.

Les compositions cosmétiques selon l'invention qui comprennent un mélange de chitosane, de glucosamine et au moins un acide organique choisi parmi l'acide gluconique et l'acide succinique, ainsi que, éventuellement, une ou plusieurs substances telles que définies ci-dessus, peuvent, bien entendu, également comporter des excipients adaptés à toutes les formes utilisées en cosmétologie : crème ou gel en pots ou en tubes, lait, émulsion pour le corps, lotion en flacon de verre ou de plastique et éventuellement en flacon doseur ou encore en ampoules, savon liquide ou pain dermatologique.

Pour chaque forme, on a recours à des excipients appropriés. Ces excipients doivent avoir toutes les qualités habituellement demandées. Ils doivent être doués d'une grande affinité pour la peau, être parfaitement bien tolérés, stables, présenter une consistance adéquate permettant une utilisation facile et agréable.

A titre d'exemples d'excipients pouvant être utilisés, on peut citer des polymères de type carboxyvinylique, les polyéthylèneglycols, le propylèneglycol, des cires, des corps gras, des esters et des triglycérides d'acides gras, des dérivés stéariques tel que le stéarate de glycérol, des alcools tels que par exemple, les alcools stéaryliques, les alcools cétostéaryliques, l'alcool cétylique,, polyol, l'éther cétylique polyoxyéthylène, des huiles végétales telles que l'huile d'amande douce, des huiles minérales telles que l'huile de vaseline, la glycérine, des dérivés de la lanoline, du talc, des mouillants, des épaississants, des stabilisants, des émulsionnants, des conservateurs, des parfums, des colorants ou d'autres excipients connus et couramment utilisés.

Des exemples de telles compositions sont donnés ci-aprés.

Les formes cosmétiques préférées sont les compositions cosmétiques telles que définies ci-dessus caractérisées en ce qu'elles se présentent sous forme de crème, de lotion tonique, d'une émulsion pour le corps, de savon liquide ou de pain dermatologique.

Par ailleurs, l'invention concerne l'application à titre de produit cosmétique de l'association de chitosane à de la glucosamine et au moins à un acide organique choisi parmi l'acide gluconique ou l'acide succinique et une méthode de traitement cosmétique de la peau sèche ou desséchée caractérisée en ce que l'on applique sur la peau une quantité suffisante d'une composition cosmétique telle que définie ci-dessus.

Les différentes formes cosmétiques mentionnées ci-dessus peuvent être obtenues selon les méthodes usuelles utilisées dans ce domaine.

Les exemples suivants illustrent l'invention.

1 - On a déterminé le taux d'hygroscopicité des mélanges définis ci-aprés.

Mélange 1 :

- chitosane            1
- acide gluconique       0,5
- acide aspartique       0,7
- glucosamine HCl       0,5
- hydroxyde de sodium       0,11

absorption d'eau dans une atmosphère à 80 % d'humidité relative : 22 %.

Mélange 2 :

- chitosane            1
- acide gluconique       0,5
- acide glutamique       0,8
- glucosamine HCl       0,5
- L-arginine         0,6

absorption d'eau dans une atmosphère à 80 % d'humidité relative : 29,8 %.

Mélange 3 :

- chitosane            1
- acide gluconique            0,5
- acide pyrrolidonecarboxylique            0,7
- glucosamine HCl            0,5
- arginine            0,6

absorption d'eau dans une atmosphère à 80 % d'humidité relative : 31 %.

2 - On a réalisé les compositions cosmétiques définies ci-aprés.

**Exemple 1: Crème hydratante.**

| | |
|---|---|
| Eau | 54 |
| Mélange hydratant 2 | 3,2 |
| Acide stéarique éthoxylé 500 E | 2,5 |
| Acide stéarique éthoxylé 100 E | 1,5 |
| Huile végétale | 3 |
| Huile minérale | 15 |
| Phytostérol | 0,8 |
| Monostéarate de glycérol | 8 |
| Myristate d'isophropyle | 10 |
| Méthoxycinnamate d'octyle | 2 |
| Conservateur | qs |
| Parfum | qs |

**Exemple 2: Crème solaire hydratante.**

| | |
|---|---|
| Octyl diméthylaminobenzoate | 5 |
| Alcool cétylique | 5 |
| Huile de sésame | 2 |
| Cire de riz | 4 |
| Cétylstéaryl éthylhéxanoate | 3 |
| Cyclomethicone D5 | 7 |
| Alcool cétylique éthoxylé 100 OE | 1 |
| Monaquat P TS$^{r}$ (Mona) | 3,5 |
| Eau | 66,2 |
| Parfum | qs |
| Conservateur | qs |
| Mélange hydratant 3 | 3,3 |

**Exemple 3 : Lotion tonique hydratante peaux grasses.**

| | |
|---|---|
| Eau | 94,35 |
| Azulène | 0,1 |
| Nicotinamide | 0,2 |
| Mélange hydratant 1 | 4,05 |
| Octyldodécanol éthoxylé 30 OE | 1,3 |
| Parfum | qs |
| Conservateur | qs |

**Exemple 4 : Crème hydratante corps.**

| | | |
|---|---|---|
| Eau | qs | 100 |
| Huile de Macadamia | | 1,2 |
| Perhydrosqualène | | 0,45 |
| Stérols de soja | | 0,30 |
| Acide linoléique | | 0,15 |
| Monolinoléate de glycérol | | 0,30 |
| Undécylénate de glycérol | | 0,60 |
| Huile de Silicone | | 2,00 |
| Hexyl décanol | | 5,00 |
| Myristate de Myristyle | | 5,00 |
| Stéarate de propylèneglycol | | 1,50 |
| Glycérine | | 3,00 |
| Acide pyrrolique carboxylique | | 0,15 |
| Stéaramidopropyl PG ammonium chlorure de phosphate | | 3,00 |
| Conservateurs | | 2,00 |
| | | |
| Mélange 1 | | 4,00 |
| Parfum | | 0,30 |

**Exemple 5 : Huile de bain**

| | |
|---|---|
| Alcool oléique polyoxyéthylène | 9 |
| Alcool laurique polyoxyéthylène | 3 |
| Glycérides d'acides caprique/caprylique P.O.E. | 8 |
| Huile de Macadamia | 1,2 |
| Perhydrosqualène | 0,45 |
| Stérols de soja | 0,30 |
| Acide linoléique | 0,15 |
| Monolinoléate de glycérol | 0,30 |
| Undécylénate de glycérol | 0,60 |
| Polyéthylène 400 | 10,00 |
| Dipropylène glycol | 21,00 |
| Eau | qs 100 |
| Lactamide MEA | 1,00 |
| Conservateur | 0,05 |
| Anti-oxydant | 0,05 |
| Monoglycéride citrate | 0,1 |
| Mélange 1 | 2,00 |
| Parfum | 0,50 |

**Exemple 6 : Gel nottoyant**

| | |
|---|---|
| Lauryl sulfate de magnésium | 2,0 |
| 3-(3-cocoamidopropyl) diméthylammonium 2-hydroxypropane | 7,5 |
| Cocoyl isothionate de sodium | 9,0 |
| Lauryl éther 2-maide MEA | 1,0 |
| Monostéarate d'éthylène glycol | 2,0 |
| PEG-200 glycéryl monotallowate | 4,5 |
| Glycérides d'acides caprique/caprylique P.O.E. | 1,0 |
| Stéramidopropyl PG ammonium chlorure phosphate | 2,0 |
| PEG-78 glycéryl mono cocoate | 0,5 |
| Eau | qs 100 |
| Conservateurs | 2,0 |
| Parfum | 0,5 |
| Mélange 2 | 4,0 |

**Revendications**

1.   Compositions cosmétiques renfermant du chitosane, de la glucosamine additionnelle et au moins un acide organique choisi parmi l'acide gluconique ou l'acide succinique, destinées notamment à hydrater et

6

améliorer l'état de surface de l'épiderme.

2. Compositions cosmétiques telles que définies à la revendication 1, caractérisées en ce que :
le chitosane est compris entre 0,2 et 5 %,
la glucosamine est comprise entre 0,2 et 5 %,
l'acide gluconique est compris entre 0,2 et 5 %,
l'acide succinique est compris entre 0,1 et 5 %.

3. Compositions cosmétiques telles que définies à la revendication 1 ou 2, caractérisées en ce que le chitosane qu'elles renferment comprend 0 à 30 % de groupements aminés acétylés.

4. Compositions cosmétiques telles que définies à l'une quelconque des revendications 1 à 3, caractérisées en ce que le chitosane qu'elles renferment provient de carapaces de crabe et comprend moins de 15 % de groupements aminés acétylés.

5. Compositions cosmétiques telles que définies à l'une quelconque des revendications 1 à 4, caractérisées en ce que le chitosane qu'elles renferment comprend de 0,1 à 5% de groupements aminés acétylés.

6. Compositions cosmétiques telles que définies à l'une quelconque des revendications 1 à 5, caractérisées en ce qu'elles renferment une ou plusieurs substances à une concentration suffisante pour amener le pH de la préparation entre 3 et 6,5.

7. Compositions cosmétiques telles que définies à l'une quelconque des revendications 1 à 6, caractérisées en ce qu'elles se présentent sous forme de crème, de lotion tonique, d'une émulsion pour le corps, de savon liquide ou de pain dermatologique.

8. Procédé de préparation des compositions telles que définies aux revendications 1 à 7, caractérisées en ce que l'on prépare d'abord une solution acide d'un pH inférieur à 3,5 en mélangeant le chitosane à au moins un des acides gluconique ou succinique puis ajoute les autres constituants de la composition.

9. Application à titre de produit cosmétique de l'association de chitosane à de la glucosamine et au moins à un acide organique choisi parmi l'acide gluconique ou l'acide succinique.

10. Méthode de traitement cosmétique de la peau sèche ou desséchée caractérisée en ce que l'on applique sur la peau une quantité suffisante d'une composition cosmétique telle que définie selon l'une quelconque des revendications 1 à 6.

## Patentansprüche

1. Kosmetische Zusammensetzungen, umfassend Chitosan, zusatzlich Glucosamin und mindestens eine organische Saure, ausgewahlt unter Gluconsäure oder Bernsteinsaure, die insbesondere zum Hydratisieren und zur Verbesserung des Oberflachenzustandes der Epidermis vorgesehen sind.

2. Kosmetische Zusammensetzungen wie in Anspruch 1 definiert, dadurch gekennzeichnet, daß
das Chitosan in einer Menge zwischen 0,2 und 5 % enthalten ist,
das Glucosamin in einer Menge zwischen 0,2 und 5 % enthalten ist,
die Gluconsäure in einer Menge zwischen 0,2 und 5 % enthalten ist,
die Bernsteinsäure in einer Menge zwischen 0,1 und 5 % enthalten ist.

3. Kosmetische Zusammensetzungen wie in Anspruch 1 oder 2 definiert, dadurch gekennzeichnet, daß das enthaltene Chitosan 0 bis 30 % acetylierte Aminogruppen umfaßt.

4. Kosmetische Zusammensetzungen wie in irgendeinem der Ansprüche 1 bis 3 definiert, dadurch gekennzeichnet, daß das enthaltene Chitosan von Krabbenpanzern stammt und weniger als 15 % acetylierte Aminogruppen umfaßt.

5. Kosmetische Zusammensetzungen wie in irgendeinem der Ansprüche 1 bis 4 definiert, dadurch gekennzeichnet, daß das enthaltene Chitosan 0,1 bis 5 % acetylierte Aminogruppen umfaßt.

**6.** Kosmetische Zusammensetzungen wie in irgendeinem der Ansprüche 1 bis 5 definiert, dadurch gekennzeichnet, daß sie eine oder mehrere Substanzen in einer Konzentration umfassen, die ausreichend ist, um den pH-Wert der Präparation zwischen 3 und 6,5 einzustellen.

**7.** Kosmetische Zusammensetzungen wie in irgendeinem der Ansprüche 1 bis 6 definiert, dadurch gekennzeichnet, daß sie in Form von Creme, tonisierender Lotion, einer Körper-Emulsion, flüssiger Seife oder einem dermatologischen Behandlungsstück vorliegen.

**8.** Verfahren zur Herstellung der Zusammensetzungen wie in Anspruch 1 bis 7 definiert, dadurch gekennzeichnet, daß man zuerst eine saure Lösung mit einem pH-Wert von unter 3,5 herstellt, indem man das Chitosan mit mindestens einer der Säuren Gluconsäure oder Bernsteinsäure mischt und anschließend die anderen Bestandteile der Zusammensetzung hinzufügt.

**9.** Anwendung der Assoziation von Chitosan mit Glucosamin und mindestens einer organischen Säure, ausgewählt unter Gluconsäure oder Bernsteinsäure, als kosmetisches Produkt.

**10.** Methode zur kosmetischen Behandlung der trockenen oder ausgetrockneten Haut, dadurch gekennzeichnet, daß man eine ausreichende Menge einer kosmetischen Zusammensetzung, wie in irgendeinem der Ansprüche 1 bis 6 definiert, auf die Haut aufbringt.

## Claims

**1.** Cosmetic compositions containing chitosan, additional glucosamine and at least one organic acid chosen from gluconic acid or succinic acid, intended in particular for moisturizing and improving the surface condition of the epidermis.

**2.** Cosmetic compositions as defined in claim 1, characterized in that:
the chitosan is comprised between 0.2 and 5%,
the glucosamine is comprised between 0.2 and 5%,
the gluconic acid is comprised between 0.2 and 5%,
the succinic acid is comprised between 0.1 and 5%.

**3.** Cosmetic compositions as defined in claim 1 or 2, characterized in that the chitosan that they contain comprises 0 to 30% acetylated amino groups.

**4.** Cosmetic compositions as defined in any one of claims 1 to 3, characterized in that the chitosan that they contain originates from crab shells and contains less than 15% acetylated amino groups.

**5.** Cosmetic compositions as defined in any one of claims 1 to 4, characterized in that the chitosan that they contain comprises 0.1 to 5% acetylated amino groups.

**6.** Cosmetic compositions as defined in any one of claims 1 to 5, characterized in that they contain one or more substances at a sufficient concentration to adjust the pH of the preparation to between 3 and 6.5.

**7.** Cosmetic compositions as defined in any one of claims 1 to 6, characterized in that they are presented in the form of a cream, toning lotion, an emulsion for the body, liquid soap or dermatological bar.

**8.** Preparation process for the compositions as defined in claims 1 to 7, characterized in that firstly, an acid solution is prepared with a pH lower than 3.5 by mixing the chitosan with at least one of the gluconic or succinic acids, then the other constituents of the composition are added.

**9.** Use as a cosmetic product of the combination of chitosan with glucosamine and at least one organic acid chosen from gluconic acid or succinic acid.

**10.** Method for the cosmetic treatment of dry or dried-out skin characterized in that a sufficient quantity of a cosmetic composition as defined according to any one of claims 1 to 6 is applied to the skin.